(19) **Europäisches Patentamt**
**European Patent Office**
**Office européen des brevets**

(11) **EP 3 581 634 B1**

(12) **FASCICULE DE BREVET EUROPEEN**

(45) Date de publication et mention
de la délivrance du brevet:
**20.01.2021 Bulletin 2021/03**

(21) Numéro de dépôt: **19177872.9**

(22) Date de dépôt: **03.06.2019**

(51) Int Cl.:
*C10G 45/64* (2006.01)  *B01J 23/40* (2006.01)
*B01J 29/06* (2006.01)  *B01J 23/08* (2006.01)
*B01J 23/14* (2006.01)  *B01J 29/064* (2006.01)
*B01J 29/72* (2006.01)  *B01J 29/74* (2006.01)
*B01J 35/00* (2006.01)  *B01J 35/02* (2006.01)
*B01J 37/18* (2006.01)  *B01J 37/20* (2006.01)
*C01B 39/48* (2006.01)  *C07C 5/27* (2006.01)
*C10G 2/00* (2006.01)  *C10G 3/00* (2006.01)
*C10G 45/62* (2006.01)

(54) **UTILISATION D'UN CATALYSEUR BIFONCTIONNEL A BASE D'IZM-2 A RAPPORT SI/AL SPECIFIQUE POUR L'ISOMERISATION DE CHARGES PARAFFINIQUES LONGUES EN DISTILLATS MOYENS**

VERWENDUNG EINES BIFUNKTIONELLEN KATALYSATORS AUF IZM-2-BASIS MIT EINEM SPEZIFISCHEN SI/AL-VERHÄLTNIS ZUR ISOMERISIERUNG VON LANGEN PARAFFINGEMISCHEN IN MITTELDESTILLATE

USE OF A BIFUNCTIONAL CATALYST MADE OF IZM-2 WITH SPECIFIC SI/AL RATIO FOR THE ISOMERISATION OF LONG PARAFFIN CHARGES INTO MEDIUM DISTILLATES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.06.2018 FR 1855286**

(43) Date de publication de la demande:
**18.12.2019 Bulletin 2019/51**

(73) Titulaire: **IFP Energies nouvelles**
**92852 Rueil-Malmaison Cedex (FR)**

(72) Inventeur: **BOUCHY, Christophe**
**69007 LYON (FR)**

(56) Documents cités:
**FR-A1- 2 934 794    FR-A1- 3 054 454**

EP 3 581 634 B1

## Description

### Domaine de l'invention

Afin de répondre à la demande en bases distillats moyens, c'est-à-dire en coupe incorporable au pool kérosène et/ou gazole, diverses méthodes de production de distillats moyens basées sur l'utilisation du pétrole, de gaz naturel ou encore de ressources renouvelables peuvent être mises en œuvre.

Les bases distillats moyens peuvent ainsi être produites à partir d'une charge paraffinique obtenue à partir d'une charge issue de sources renouvelables, et en particulier d'huiles végétales ou des graisses animales, brutes ou ayant subi un traitement préalable, ainsi que les mélanges de telles charges. En effet, lesdites charges issues de sources renouvelables contiennent des structures chimiques de type triglycérides ou esters ou acides gras libres, la structure et la longueur de chaîne hydrocarbonée de ces derniers étant compatibles avec les hydrocarbures présents dans les distillats moyens. Lesdites charges issues de sources renouvelables produisent, après hydrotraitement, des charges paraffiniques, exemptes de composés soufrés et de composés aromatiques. Ces charges paraffiniques sont typiquement composées de paraffines linéaires ayant un nombre d'atomes de carbone compris entre 9 et 25.

Les bases distillats moyens peuvent aussi être produites à partir de gaz naturel, charbon, ou sources renouvelables par l'intermédiaire du procédé de synthèse de Fischer-Tropsch. En particulier la synthèse de Fischer-Tropsch dite basse température utilisant des catalyseurs au cobalt permet de produire des composés essentiellement paraffiniques linéaires ayant un nombre d'atomes de carbone très variable, typiquement de 1 à 100 atomes de carbone voire plus. Des étapes de séparation peuvent permettre de récupérer des charges paraffiniques ayant un nombre d'atomes de carbone compris entre 9 et 25.

Toutefois, ces bases distillats moyens obtenues après hydrotraitement des huiles végétales ou après le procédé de synthèse de Fischer-Tropsch basse température ne peuvent généralement pas être incorporées telles quelles au pool kérosène ou gazole notamment en raison de propriétés à froid insuffisantes. En effet, les paraffines de haut poids moléculaire qui sont linéaires ou très faiblement branchées et qui sont présentes dans ces bases distillats moyens conduisent à des points d'écoulement hauts et donc à des phénomènes de figeage pour des utilisations à basse température. Par exemple, le point d'écoulement d'un hydrocarbure linéaire contenant 20 atomes de carbone par molécule et dont le température d'ébullition égale à 340°C environ c'est à dire typiquement comprise dans la coupe distillats moyens, est de +37°C environ ce qui rend son utilisation impossible, la spécification étant de -15°C pour le gazole. Afin de diminuer les valeurs des points d'écoulement, ces paraffines linéaires ou très peu branchées doivent être entièrement ou partiellement éliminées.

Cette opération peut s'effectuer par extraction par des solvants tels que le propane ou la méthyl-éthyl cétone, on parle alors de déparaffinage au propane ou à la méthyl éthyl-cétone (MEK). Cependant, ces techniques sont coûteuses, longues et pas toujours aisées à mettre en œuvre.

Le craquage sélectif des chaînes paraffiniques linéaires les plus longues qui conduit à la formation de composés de poids moléculaire plus faible dont une partie peut être éliminée par distillation constitue une solution pour diminuer les valeurs des points d'écoulement. Compte tenu de leur sélectivité de forme les zéolithes sont parmi les catalyseurs les plus utilisés pour ce type de procédé. Le catalyseur le plus utilisé dans la catégorie déparaffinage par craquage sélectif est la zéolithe ZSM-5, de type structural MFI, qui présente une porosité tridimensionnelle, avec des pores moyens (ouverture à 10 atomes d'oxygènes 10MR). Toutefois, le craquage occasionné dans de tels procédés conduit à la formation de quantités importantes de produits de poids moléculaires plus faibles, tels que du butane, propane, éthane et méthane, ce qui réduit considérablement le rendement en produits recherchés.

Une autre solution pour améliorer la tenue à froid consiste à isomériser les paraffines linéaires longues en minimisant au maximum le craquage. Ceci peut être réalisé par la mise en œuvre de procédé d'hydroisomérisation employant des catalyseurs bifonctionnels. Les catalyseurs bifonctionnels mettent en jeu une phase acide de Bronsted (par exemple une zéolithe) et une phase hydro/déshydrogénante (par exemple du platine) et généralement une matrice (par exemple de l'alumine). Le choix approprié de la phase acide permet de favoriser l'isomérisation des paraffines linéaires longues et de minimiser le craquage. Ainsi la sélectivité de forme des zéolithes monodimensionnelles à pores moyens (10MR) comme les zéolithes ZSM-22, ZSM-23, NU-10, ZSM-48, ZBM-30 rend leur utilisation particulièrement adaptée pour obtenir des catalyseurs sélectifs envers l'isomérisation. Ces exemples illustrent la recherche continue effectuée pour développer des catalyseurs toujours plus performants pour l'isomérisation des paraffines linéaires longues, en minimisant la formation de produits de craquage par la mise en œuvre de zéolithes appropriées.

En plus de la sélectivité envers l'isomérisation, l'activité du catalyseur est également un paramètre important. Augmenter l'activité du catalyseur permet d'améliorer le fonctionnement global du procédé du point de vue de sa productivité ou de sa consommation énergétique. Il est donc souhaitable de développer des catalyseurs les plus actifs et les plus sélectifs possibles envers l'isomérisation. L'activité des catalyseurs bifonctionnels d'isomérisation est pour une large part dépendante de l'activité de la phase acide de Bronsted (par exemple une zéolithe), et donc de son acidité, mise en œuvre dans lesdits catalyseurs. L'acidité de la phase zéolithique est in fine fonction du nombre de sites acides de Bronsted de ladite phase et également de leur force (C. Marcilly, catalyse acido-basique, volume 1, 2003). Un moyen pour augmenter l'activité d'un catalyseur bifonctionnel d'isomérisation peut donc être d'augmenter l'acidité de la phase zéolithique engagée dans ledit catalyseur en augmentant le densité de sites acides de la phase zéolithique, toutes choses égales par ailleurs.

Il est cependant également bien connu que pour un catalyseur bifonctionnel d'isomérisation, le rapport entre le nombre de sites de la phase hydro/déshydrogénante et le nombre de sites de la phase acide a un impact sur sa sélectivité en isomérisation. Une diminution trop importante de ce rapport entraine une diminution de la sélectivité en isomérisation du catalyseur. Ceci a par exemple été reporté pour l'isomérisation du n-décane sur des catalyseurs à base de platine et de zéolithe USY (F. Alvarez et coll. Journal of Catalysis, 162, 1996, 179). Plus récemment cela a également été reporté pour l'isomérisation du n-hexadécane sur des catalyseurs à base de platine, de zéolithe USY ou de zéolithe BEA (P. Mendes et coll., AIChE Journal, 63, 7, 2017, 2864).

L'augmentation de l'activité du catalyseur bifonctionnel et la maximisation de sa sélectivité en isomérisation requiert donc des exigences contradictoires en termes de densité de sites acides pour la zéolithe engagée dans le catalyseur.

Récemment, la demanderesse dans ses travaux a mis au point une nouvelle zéolithe, la zéolithe IZM-2 telle que décrite dans la demande FR 2 918 050 A, ainsi qu'un procédé de conversion de charges paraffiniques longues ayant un nombre d'atomes de carbone compris entre 9 et 25 mettant en œuvre un catalyseur comprenant ladite zéolite IZM-2 tel que décrit dans la demande de brevet FR 2 984 911A, ledit procédé permettant d'améliorer la sélectivité envers la production en base distillats moyens en limitant la production de produits craqués légers ne pouvant pas être incorporés dans un pool gazole et/ou kérosène.

FR 2 918 050 B divulgue un solide IZM-2 présentant un rapport molaire global silicium sur aluminium Si/Al compris entre 1 et l'infini, et de préférence entre 25 et 312. Dans l'exemple illustratif de la demande de brevet FR 2 984 911 A, seul un solide IZM-2 présentant un rapport molaire global Si/Al de 53 est utilisé dans la formulation du catalyseur. Ce rapport molaire global Si/Al a été calculé à partir des résultats de caractérisation par fluorescence X. FR 2 934 794 divulgue un procédé de production de distillats moyens.

Les travaux de recherche effectués par le demandeur l'ont conduit à découvrir que, de façon surprenante, l'utilisation d'un catalyseur à base d'une zéolithe IZM-2 ayant un rapport molaire Si/Al optimisé dans un procédé d'isomérisation d'une charge paraffinique permet d'améliorer l'activité du catalyseur tout en conservant une sélectivité en isomérisation maximale.

En particulier, l'utilisation d'un tel catalyseur dans le procédé d'isomérisation selon l'invention conduit à des performances catalytiques optimales en terme d'activité et de sélectivité. Un tel catalyseur est sensiblement plus sélectif qu'un catalyseur comprenant une zéolithe IZM-2 dont le rapport molaire Si/Al est inférieur à 25 et il est sensiblement plus actif qu'un catalyseur comprenant une zéolithe IZM-2 dont le rapport molaire Si/Al est supérieur à 55.

Un objet de la présente invention concerne un procédé d'isomérisation de charges paraffiniques, de préférence issues des huiles végétales et/ou animales hydrotraitées ou de la synthèse Fischer-Trospch basse température, ledit procédé mettant en œuvre un catalyseur bifonctionnel comprenant au moins un métal du groupe VIII de la classification périodique des éléments, au moins une matrice et au moins une zéolithe IZM-2, dans lequel le rapport entre le nombre de moles de silicium et le nombre de moles d'aluminium du réseau de la zéolithe IZM-2 est compris entre 25 et 55.

**Objet de l'invention**

La présente invention concerne un procédé comme défini dans la revendication 1.

Au sens de la présente invention on entend par rapport entre le nombre de moles de silicium et le nombre de moles d'aluminium de la zéolithe IZM-2 le rapport du nombre de moles de silicium divisé par le nombre de moles d'aluminium du réseau de la zéolithe IZM-2. Les atomes d'aluminium présents dans le solide mais non intégrés dans la charpente de la zéolithe (aluminium extra réseau) ne sont donc pas pris en compte dans le calcul.

Un avantage de la présente invention est de fournir un procédé d'isomérisation d'une charge paraffinique utilisant un catalyseur à base d'une zéolithe IZM-2 ayant un rapport molaire Si/Al optimisé permettant d'améliorer l'activité du catalyseur tout en conservant une sélectivité en isomérisation maximale.

**Description détaillée de l'invention**

Conformément à l'invention, la présente invention concerne un procédé comme défini dans la revendication 1.

**Le procédé d'isomérisation**

Conformément à l'invention, le procédé d'isomérisation est mis en œuvre à une température comprise entre 200°C et 500°C, à une pression totale comprise entre 0,45 MPa et 7 MPa, à une pression partielle d'hydrogène comprise entre 0,3 et 5,5 MPa, à une vitesse spatiale horaire comprise entre 0,1 et 10 kilogramme de charge introduite par kilogramme de catalyseur et par heure. De préférence, ledit procédé est effectué à une température comprise entre 200 et 450°C, et de manière plus préférée entre 220 et 430°C, à une pression totale comprise entre 0,6 et 6 MPa, à une pression partielle d'hydrogène comprise entre 0,4 et 4,8 MPa, à une vitesse spatiale horaire avantageusement comprise entre 0,2 et 7 $h^{-1}$ et de manière préférée, entre 0,5 et 5 $h^{-1}$.

Selon l'invention, le procédé d'isomérisation comprend la mise en contact d'une charge paraffinique avec au moins ledit catalyseur selon l'invention présent dans un réacteur catalytique.

Les paraffines de ladite charge paraffinique présentent un nombre d'atomes de carbone compris entre 9 et 25, de préférence compris entre 10 et 25 et de manière très préférée entre 10 et 22. La teneur en paraffines dans ladite charge mise en œuvre dans le procédé selon l'invention est avantageusement supérieure à 90% poids, de préférence supérieure à 95% poids, de manière encore plus préférée supérieure à 98% poids. Au sein desdites parrafines, le pourcentage massique d'isoparaffines est inférieur à 15%, de manière préférée inférieur à 10% et de manière très préférée inférieur à 5%.

Selon un premier mode de réalisation, ladite charge paraffinique utilisée dans le procédé selon l'invention peut être produite à partir de ressources renouvelables.

De préférence, ladite charge paraffinique est produite à partir de ressources renouvelables choisies parmi les huiles végétales, les huiles d'algues ou algales, les huiles de poissons et les graisses d'origine végétale ou animale, ou des mélanges de telles charges.

Lesdites huiles végétales peuvent avantageusement être brutes ou raffinées, totalement ou en partie, et issues des végétaux choisis parmi le colza, le tournesol, le soja, le palmier, l'olive, la noix de coco, le coprah, le ricin, le coton, les huiles d'arachides, de lin et de crambe et toutes les huiles issues par exemple du tournesol ou du colza par modification génétique ou hybridation, cette liste n'étant pas limitative. Lesdites graisses animales sont avantageusement choisies parmi le lard et les graisses composées de résidus de l'industrie alimentaire ou issus des industries de la restauration. Les huiles de fritures, les huiles animales variées comme les huiles de poisson, le suif, le saindoux peuvent également être utilisées.

Les ressources renouvelables à partir desquelles est produite la charge paraffinique utilisé dans le procédé selon l'invention contiennent essentiellement des structures chimiques de type triglycérides que l'homme du métier connait également sous l'appellation tri ester d'acides gras ainsi que des acides gras libres, dont les chaînes grasses contiennent un nombre d'atomes de carbone compris entre 9 et 25.

La structure et la longueur de chaîne hydrocarbonée de ces derniers est compatible avec les hydrocarbures présents dans le gazole et le kérosène, c'est à dire la coupe distillats moyens. Un tri ester d'acide gras est ainsi composé de trois chaînes d'acides gras. Ces chaînes d'acide gras sous forme de tri ester ou sous forme d'acide gras libres, possèdent un nombre d'insaturations par chaîne, également appelé nombre de doubles liaisons carbone-carbone par chaîne, généralement compris entre 0 et 3 mais qui peut être plus élevé notamment pour les huiles issues d'algues qui présentent généralement un nombre d'insaturations par chaînes de 5 à 6.

Les molécules présentes dans lesdites ressources renouvelables utilisées dans la présente invention présentent donc un nombre d'insaturations, exprimé par molécule de triglycéride, avantageusement compris entre 0 et 18. Dans ces charges, le taux d'insaturation, exprimé en nombre d'insaturations par chaîne grasse hydrocarbonée, est avantageusement compris entre 0 et 6.

Les ressources renouvelables comportent généralement également différentes impuretés et notamment des hétéroatomes tels que l'azote. Les teneurs en azote dans les huiles végétales sont généralement comprises entre 1 ppm et 100 ppm poids environ, selon leur nature. Elles peuvent atteindre jusqu'à 1 % poids sur des charges particulières.

Ladite charge paraffinique utilisée dans le procédé selon l'invention est avantageusement produite à partir de ressources renouvelables selon des procédés connus de l'homme du métier. Une voie possible est la transformation catalytique desdites ressources renouvelables en effluent paraffinique désoxygéné en présence d'hydrogène et en particulier, l'hydrotraitement.

De préférence, ladite charge paraffinique est produite par hydrotraitement desdites ressources renouvelables. Ces procédés d'hydrotraitement de ressources renouvelables sont déjà bien connus et sont décrits dans de nombreux brevets. A titre d'exemple, ladite charge paraffinique utilisée dans le procédé selon l'invention peut avantageusement être produite, de préférence par hydrotraitement puis par séparation gaz/liquide, à partir desdites ressources renouvelables comme par exemple dans le brevet FR 2 910 483 ou dans le brevet FR 2 950 895.

Selon un deuxième mode de réalisation, ladite charge paraffinique utilisée dans le procédé selon l'invention peut aussi être une charge paraffinique produite par un procédé mettant en jeu une étape de valorisation par la voie Fischer-Tropsch. Dans le procédé Fischer-Tropsch, le gaz de synthèse ($CO+H_2$) est transformé catalytiquement en produits oxygénés et en hydrocarbures essentiellement linéaires sous forme gazeuse, liquide ou solide. Lesdits produits obtenus constituent la charge du procédé selon l'invention. Le gaz de synthèse ($CO+H_2$) est avantageusement produit à partir de gaz naturel, de charbon, de biomasse, de toute source de composés hydrocarbonés ou d'un mélange de ces sources. Ainsi, les charges paraffiniques obtenues, selon un procédé de synthèse Fischer-Tropsch, à partir d'un gaz de synthèse (CO+H$_2$) produit à partir de ressources renouvelables, de gaz naturel ou de charbon peuvent être utilisées dans le procédé selon l'invention. De préférence, ladite charge paraffinique produite par synthèse Fischer-Tropsch et utilisée dans le procédé selon l'invention comprend majoritairement des n-paraffines. Ainsi, ladite charge comprend une teneur en n-paraffines supérieure à 60% poids par rapport à la masse totale de ladite charge. Ladite charge peut également comprendre une teneur en produits oxygénés de préférence inférieure à 10% poids, une teneur en insaturés, c'est-à-dire de préférence en produits oléfiniques, de préférence inférieure à 20% en poids et une teneur en iso-paraffines de préférence inférieure à 10% en poids par rapport à la masse totale de ladite charge.

La charge comprend une teneur en n-paraffines supérieure à 70% poids et de manière encore plus préférée supérieure à 80% poids par rapport à la masse totale de ladite charge. Les paraffines de ladite charge paraffinique présentent un nombre d'atomes de carbone compris entre 9 et 25, de préférence compris entre 10 et 25 et de manière très préférée entre 10 et 22.

De préférence, ladite charge paraffinique produite par synthèse Fischer-Tropsch est exempte d'impuretés hétéroatomiques telles que, par exemple, le soufre, l'azote ou des métaux.

## Catalyseur

Conformément à l'invention, le catalyseur mis en œuvre dans ledit procédé comprend au moins un métal du groupe VIII de la classification périodique des éléments, au moins une matrice et au moins une zéolithe IZM-2, dans lequel le rapport entre le nombre de moles de silicium et le nombre de moles d'aluminium du réseau de la zéolithe IZM-2 est compris entre 25 et 55, de préférence entre 25 et 50 et de manière préférée entre 30 et 50.

## Zéolithe IZM-2

Conformément à l'invention, le catalyseur comprend la zéolithe IZM-2. La zéolithe IZM-2 est un solide microporeux cristallisé présentant une structure cristalline décrite dans la demande de brevet FR 2 918 050. La zéolithe IZM-2 présente un diagramme de diffraction de rayons X incluant au moins les raies inscrites dans le tableau 1.

Avantageusement, le diagramme de diffraction est obtenu par analyse radiocristallographique au moyen d'un diffractomètre en utilisant la méthode classique des poudres avec le rayonnement $K_{\alpha 1}$ du cuivre ($\lambda$ = 1,5406 Å). A partir de la position des pics de diffraction représentée par l'angle $2\theta$, on calcule, par la relation de Bragg, les équidistances réticulaires $d_{hkl}$ caractéristiques de l'échantillon. L'erreur de mesure $\Delta(d_{hkl})$ sur $d_{hkl}$ est calculée grâce à la relation de Bragg en fonction de l'erreur absolue $\Delta(2\theta)$ affectée à la mesure de $2\theta$. Une erreur absolue $\Delta(2\theta)$ égale à $\pm$ 0,02° est communément admise. L'intensité relative $I_{rel}$ affectée à chaque valeur de $d_{hkl}$ est mesurée d'après la hauteur du pic de diffraction correspondant. Le diagramme de diffraction des rayons X de IZM-2 selon l'invention comporte au moins les raies aux valeurs de $d_{hkl}$ données dans le tableau 1. Dans la colonne des $d_{hkl}$, on indique les valeurs moyennes des distances inter-réticulaires en Angströms (Å). Chacune de ces valeurs doit être affectée de l'erreur de mesure $\Delta(d_{hkl})$ comprise entre $\pm$ 0,6Å et $\pm$ 0,01 Å.

Tableau 1: valeurs moyennes des $d_{hkl}$ et intensités relatives mesurées sur un diagramme de diffraction de rayons X du solide cristallisé IZM-2 calciné.

| 2 thêta (°) | $d_{hkl}$ (Å) | $I_{rel}$ | 2 thêta (°) | $d_{hkl}$ (Å) | $I_{rel}$ |
|---|---|---|---|---|---|
| 5,07 | 17,43 | ff | 19,01 | 4,66 | ff |
| 7,36 | 12,01 | FF | 19,52 | 4,54 | ff |
| 7,67 | 11,52 | FF | 21,29 | 4,17 | m |
| 8,78 | 10,07 | F | 22,44 | 3,96 | f |
| 10,02 | 8,82 | ff | 23,10 | 3,85 | mf |
| 12,13 | 7,29 | ff | 23,57 | 3,77 | f |
| 14,76 | 6,00 | ff | 24,65 | 3,61 | ff |
| 15,31 | 5,78 | ff | 26,78 | 3,33 | f |
| 15,62 | 5,67 | ff | 29,33 | 3,04 | ff |
| 16,03 | 5,52 | ff | 33,06 | 2,71 | ff |
| 17,60 | 5,03 | ff | 36,82 | 2,44 | ff |

(suite)

| 2 thêta (°) | $d_{hkl}$ (Å) | $I_{rel}$ | 2 thêta (°) | $d_{hkl}$ (Å) | $I_{rel}$ |
|---|---|---|---|---|---|
| 18,22 | 4,87 | ff | 44,54 | 2,03 | ff |
| où FF = très fort ; F = fort ; m = moyen ; mf = moyen faible ; f = faible ; ff = très faible. | | | | | |

L'intensité relative $I_{rel}$ est donnée en rapport à une échelle d'intensité relative où il est attribué une valeur de 100 à la raie la plus intense du diagramme de diffraction des rayons X : ff < 15 ; 15 ≤ f < 30 ; 30 ≤ mf < 50 ; 50 ≤ m < 65 ; 65 ≤ F < 85 ; FF ≥ 85.

IZM-2 présente une composition chimique exprimée sur une base anhydre, en termes de moles d'oxydes, définie par la formule générale suivante : $SiO_2$ : a $Al_2O_3$ : b $M_{2/n}O$, dans laquelle M est au moins un métal alcalin et/ou un métal alcalino-terreux de valence n. Dans ladite formule donnée ci-dessus, a représente le nombre de moles de $Al_2O_3$ et b représente le nombre de moles de $M_{2/n}O$.

Conformément à l'invention, ledit catalyseur mis en œuvre dans ledit procédé comprend au moins une zéolithe IZM-2, dans lequel le rapport entre le nombre de moles de silicium et le nombre de moles d'aluminium du réseau de la zéolithe IZM-2 est compris entre 25 et 55, de préférence entre 25 et 50 et de manière préférée entre 30 et 50.

Selon l'invention, le rapport du nombre de moles de silicium divisé par le nombre de moles d'aluminium de réseau de la zéolithe IZM-2 (Si/Al) est calculé selon la formule :

$$Si/Al = n_{Si} / n_{Al}$$

avec

$n_{Si} / n_{Al}$ : rapport du nombre de moles de silicium divisé par le nombre de moles d'aluminium de réseau, en mole/mole,
$n_{Si}$ : moles de silicium par gramme de la zéolithe, en mole/gramme,
$n_{Al}$ : moles d'aluminium de réseau par gramme de zéolithe, en mole/gramme.

Selon l'invention, le nombre de moles d'aluminium de réseau par gramme de zéolithe IZM-2 se détermine à partir du pourcentage (%) poids (pds) en aluminium de la zéolithe et du pourcentage d'aluminium tétracoordinés et pentacoordinés présent dans la zéolithe selon la formule :

$$n_{Al} = [(\%pdsAl) * (\%RMNAl^{IV} + \%RMNAl^{V})] / [MM(Al) * 10000]$$

avec

$n_{Al}$ : moles d'aluminium de réseau par gramme de zéolithe, en mole/gramme,
%pdsAl : pourcentage poids d'aluminium dans la zéolithe (masse sèche), mesuré par plasma à couplage inductif (ICP) sur un appareil SPECTRO ARCOS ICP-OES de SPECTRO selon la méthode ASTM D7260,
MM(Al) : masse molaire de l'aluminium, en gramme/mole,
$\%RMNAl^{IV}$ : pourcentage pondéral d'aluminium tétracoordiné dans la zéolithe, mesuré par résonance magnétique nucléaire de $^{27}$Al,
$\%RMNAl^{V}$ : pourcentage pondéral d'aluminium pentacoordiné dans la zéolithe, mesuré par résonance magnétique nucléaire de $^{27}$ Al.

Les atomes d'aluminium tétracoordinés et pentacoordinés sont considérés comme des aluminium de réseau, c'est-à-dire comme des aluminium intégrés dans la charpente de la zéolithe.

Le pourcentage pondéral des atomes d'aluminium tétracoordinés et pentacoordinés présents dans la zéolithe IZM-2 est déterminé par résonance magnétique nucléaire du solide de $^{27}$ Al. La RMN de l'aluminium est en effet connue pour être utilisée en vue de repérer et de quantifier les différents états de coordination de ce noyau ("Analyse physico-chimiques des catalyseurs industriels", J. Lynch, Editions Technip (2001) chap. 13, pages 290 et 291). Le spectre RMN de l'aluminium de la zéolithe IZM-2 peut présenter trois signaux, un premier étant caractéristique de la résonance des atomes d'aluminium tétracoordinés, un second étant caractéristique des atomes d'aluminium pentacoordinés et un troisième étant caractéristique de la résonance des atomes d'aluminium hexacoordinés. Les atomes d'aluminium hexacoordinés sont considérés comme des aluminium extra réseau. Les atomes d'aluminium tétracoordinés (noté $Al^{IV}$)

résonnent à un déplacement chimique typiquement compris entre +50 ppm et +70 ppm, les atomes d'aluminium pentacoordinés (noté $Al^V$) résonnent à un déplacement chimique typiquement compris entre +20 ppm et +40 ppm et les atomes d'aluminium hexacoordinés (noté $Al^{VI}$) résonnent à un déplacement chimique typiquement compris entre -20 ppm et +10 ppm. Le pourcentage pondéral des différentes espèces aluminiques est quantifié à partir de l'intégration des signaux correspondant à chacune de ces espèces.

Plus précisément, la zéolithe IZM-2 présente dans le catalyseur selon l'invention a été analysée par RMN-MAS du solide $^{27}Al$ sur un spectromètre Brücker de type Avance 400 MHz à l'aide d'une sonde 4 mm optimisée pour $^{27}Al$. La vitesse de rotation de l'échantillon est voisine de 12 kHz. L'atome d'aluminium est un noyau quadripolaire dont le spin est égal à 5/2. Dans des conditions d'analyse dites sélectives, à savoir un champ de radiofréquence faible égal à 30 kHz, un angle d'impulsion faible égal à $\pi/2$ et en présence d'un échantillon saturé en eau, la technique de RMN de rotation à l'angle magique (MAS), notée RMN-MAS, est une technique quantitative. La décomposition de chaque spectre RMN-MAS permet d'accéder directement à la quantité des différentes espèces aluminiques, à savoir des atomes d'aluminium tétracoordinés $Al^{IV}$, pentacoordinés $Al^V$ et hexacoordinés $Al^{VI}$. Chaque spectre est calé en déplacement chimique par rapport à une solution molaire de nitrate d'aluminium pour laquelle le signal d'aluminium est à zéro ppm. Les signaux caractérisant les atomes d'aluminium tétracoordinés $Al^{IV}$ sont intégrés typiquement entre +50 ppm et +70 ppm ce qui correspond à l'aire 1, les signaux caractérisant les atomes d'aluminium pentacoordinés $Al^V$ sont intégrés typiquement entre +20 ppm et +40 ppm ce qui correspond à l'aire 2 et les signaux caractérisant les atomes d'aluminium hexacoordinés $Al^{VI}$ sont intégrés typiquement entre -20 ppm et +10 ppm ce qui correspond à l'aire 3. Le pourcentage pondéral de chaque espèce aluminique est calculé à partir du rapport entre son aire et l'aire totale. Par exemple le pourcentage pondéral d'atomes d'aluminium hexacoordinés $Al^{VI}$ (noté $\%RMNAl^{VI}$) est calculé comme suit :

$$\%RMNAl^{VI} = (aire\ 3) * 100 / (aire\ 1 + aire\ 2 + aire\ 3)$$

Avantageusement, la zéolithe IZM-2 selon l'invention présente un pourcentage pondéral d'atomes d'aluminium hexacoordinés $Al^{VI}$ (noté $\%RMNAl^{VI}$) inférieur à 50%, de manière préférée inférieur à 40%, et de manière très préférée inférieur à 30%.

Le nombre de moles de silicium par gramme de zéolithe IZM-2 se détermine à partir du pourcentage (%) poids (pds) en silicium de la zéolithe selon la formule :

$$n_{Si} = [(\%pdsSi) / [MM(Si) * 100]$$

avec

$n_{Si}$ : moles de silicium par gramme de zéolithe, en mole/gramme,

%pdsSi : pourcentage poids d'aluminium dans la zéolithe (masse sèche), mesuré par Fluorescence X par dosage en perle sur un appareil AXIOS de marque PANalytical travaillant à 125 mA and 32 kV,

MM(Si) : masse molaire du silicium, en gramme/mole.

Le rapport molaire Si/Al désiré selon l'invention pour la zéolithe IZM-2 peut être obtenu directement lors de l'étape de synthèse de la zéolithe par ajustement des conditions de synthèses et notamment de la composition du gel de synthèse en contrôlant par exemple les quantités relatives de silicium et d'aluminium engagées dans le gel de synthèse.

Un procédé de préparation de la zéolithe IZM-2 est enseigné dans la demande de brevet FR 2 918 050 A.

De manière avantageuse, on fait réagir un mélange aqueux comportant au moins une source d'au moins un oxyde $SiO_2$, éventuellement au moins une source d'au moins un oxyde $Al_2O_3$, éventuellement au moins une source d'au moins un métal alcalin et/ou alcalino-terreux de valence n, et de préférence au moins une espèce organique R comportant deux atomes d'azote quaternaires, le mélange présentant préférentiellement la composition molaire suivante :

$SiO_2/Al_2O_3$ : au moins 2, de préférence au moins 20, de manière plus préférée de 60 à 600, $H_2O/SiO_2$: 1 à 100, de préférence de 10 à 70,

$R/SiO_2$: 0,02 à 2, de préférence de 0,05 à 0,5,

$M_{2/n}O/SiO_2$: 0 à 1, de préférence de 0,005 et 0,5,

où M est un ou plusieurs métal(aux) alcalin(s) et/ou alcalino-terreux choisi(s) parmi le lithium, le sodium, le potassium, le calcium, le magnésium et le mélange d'au moins deux de ces métaux, de préférence M est le sodium. Avantageusement, l'élément R est le 1,6-bis(méthylpiperidinium)hexane.

Conformément à l'invention, le rapport molaire Si/Al de la zéolithe IZM-2 peut être aussi ajusté à la valeur désirée par des méthodes de post traitement de la zéolithe IZM-2 obtenue après synthèse. De telles méthodes sont connues de l'homme du métier, et permettent d'effectuer de la désalumination ou de la désilication de la zéolithe. De manière préférée le rapport molaire Si/Al de la zéolithe IZM-2 entrant dans la composition du catalyseur selon l'invention est ajusté par un choix approprié des conditions de synthèse de ladite zéolithe.

La zéolithe IZM-2 présente dans le catalyseur selon l'invention, se trouve très avantageusement sous sa forme acide c'est-à-dire sous forme protonée $H^+$. Dans un tel cas, il est avantageux que le rapport du nombre de moles de cation autre que le proton par gramme de zéolithe IZM-2 divisé par le nombre de moles d'aluminium de réseau par gramme de zéolithe IZM-2 soit inférieur à 0,9, de préférence inférieur à 0,6 et de manière très préférée inférieur à 0,3. Pour ce faire, la zéolithe IZM-2 entrant dans la composition du catalyseur selon l'invention peut être par exemple échangée par au moins un traitement par une solution d'au moins un sel d'ammonium de manière à obtenir la forme ammonium de la zéolithe IZM-2 qui une fois calcinée conduit à la forme acide de ladite zéolithe IZM-2. Cette étape d'échange peut être effectuée à toute étape de la préparation du catalyseur, c'est-à-dire après l'étape de préparation de la zéolithe IZM-2, après l'étape de mise en forme de la zéolithe IZM-2 avec une matrice, ou encore après l'étape d'introduction du métal hydro-déshydrogénant. De préférence l'étape d'échange est effectuée après l'étape de mise en forme de la zéolithe IZM-2.

## Matrice

Conformément à l'invention, ledit catalyseur comprend au moins une matrice. Ladite matrice peut avantageusement être amorphe ou cristallisée.

De préférence, ladite matrice est avantageusement choisie dans le groupe formé par l'alumine, la silice, la silice-alumine, les argiles, l'oxyde de titane, l'oxyde de bore et la zircone, pris seuls ou en mélange ou bien on peut choisir également les aluminates. De préférence, l'alumine est utilisée comme matrice. De manière préférée, ladite matrice contient de l'alumine sous toutes ses formes connues de l'homme du métier, telles que par exemple les alumines de type alpha, gamma, êta, delta. Lesdites alumines diffèrent par leur surface spécifique et leur volume poreux. Le mélange de la matrice et de la zéolithe IZM-2 mis en forme constitue le support du catalyseur.

## Phase métallique

Conformément à l'invention, le catalyseur comprend au moins un métal du groupe VIII de préférence choisi parmi le fer, le cobalt, le nickel, le ruthénium, le rhodium, le palladium, l'osmium, l'iridium et le platine, de préférence choisi parmi les métaux nobles du groupe VIII, de manière très préférée choisi parmi le palladium et le platine et de manière encore plus préférée on choisit le platine.

De préférence, ledit catalyseur comprend une teneur en métal du groupe VIII comprise entre 0,01 et 5% poids par rapport à la masse totale dudit catalyseur et de préférence comprise entre 0,1 et 4 % poids.

Dans le cas où ledit catalyseur comprend au moins un métal noble du groupe VIII, la teneur en métal noble dudit catalyseur est avantageusement comprise entre 0,01 et 5% en poids de manière préférée entre 0,1 et 4% en poids et de manière très préférée entre 0,1 et 2% en poids par rapport à la masse totale dudit catalyseur.

Le catalyseur de l'invention peut également avantageusement contenir au moins un métal choisi parmi les métaux des groupes IIIA, IVA et VIIB choisis parmi le gallium, l'indium, l'étain et le rhénium. Dans ce cas, la teneur en métal choisi parmi les métaux des groupes IIIA, IVA et VIIB est de préférence comprise entre 0,01 et 2%, de préférence entre 0,05 et 1% poids par rapport à la masse totale dudit catalyseur.

La dispersion du(es) métal(ux) du groupe VIII, déterminée par chimisorption, par exemple par titration $H_2/O_2$ ou par chimisorption du monoxyde de carbone, est comprise entre 10% et 100%, de préférence entre 20% et 100% et de manière encore plus préférée entre 30% et 100%. Le coefficient de répartition macroscopique du(es) métal(ux) du groupe VIII, obtenu à partir de son (leur) profil déterminé par microsonde de Castaing, défini comme le rapport des concentrations du(es) métal(ux) du groupe VIII au cœur du grain par rapport au bord de ce même grain, est compris entre 0,7 et 1,3, de préférence entre 0,8 et 1,2. La valeur de ce rapport, voisine de 1, témoigne de l'homogénéité de la répartition du(es) métal(ux) du groupe VIII dans le catalyseur.

Ledit catalyseur peut comprendre avantageusement au moins un métal additionnel choisi dans le groupe formé par les métaux des groupes IIIA, IVA et VIIB de la classification périodique des éléments et de préférence choisi parmi le gallium, l'indium, l'étain et le rhénium. Ledit métal additionnel est de préférence choisi parmi l'indium, l'étain et le rhénium.

**Préparation du catalyseur**

Le catalyseur selon l'invention peut avantageusement être préparé selon toutes les méthodes bien connues de l'homme du métier.

Mise en forme

Avantageusement, les différents constituants du support ou du catalyseur peuvent être mis en forme par étape de malaxage pour former une pâte puis extrusion de la pâte obtenue, ou alors par mélange de poudres puis pastillage, ou alors par tout autre procédé connu d'agglomération d'une poudre contenant de l'alumine. Les supports ainsi obtenus peuvent se présenter sous différentes formes et dimensions. De manière préférée la mise en forme est effectuée par malaxage et extrusion.

Lors de la mise en forme du support par malaxage puis extrusion, ladite zéolithe IZM-2 peut être introduite au cours de la mise en solution ou en suspension des composés d'alumine ou précurseurs d'alumine tels que la boéhmite par exemple. Ladite zéolithe IZM-2 peut être, sans que cela soit limitatif, par exemple sous forme de poudre, poudre broyée, suspension, suspension ayant subi un traitement de désagglomération. Ainsi, par exemple, ladite zéolithe peut avantageusement être mise en suspension acidulée ou non à une concentration ajustée à la teneur finale en IZM-2 visée dans le catalyseur selon l'invention. Cette suspension appelée couramment une barbotine est alors mélangée avec les composés d'alumine ou précurseurs d'alumine.

Par ailleurs, l'utilisation d'additifs peut avantageusement être mise en œuvre pour faciliter la mise en forme et/ou améliorer les propriétés mécaniques finales des supports comme cela est bien connu par l'homme du métier. A titre d'exemple d'additifs, on peut citer notamment la cellulose, la carboxyméthyl-cellulose, la carboxy-éthyl-cellulose, du tall-oil (huile de tall), les gommes xanthaniques, des agents tensio-actifs, des agents floculants comme les polyacrylamides, le noir de carbone, les amidons, l'acide stéarique, l'alcool polyacrylique, l'alcool polyvinylique, des biopolymères, le glucose, les polyéthylènes glycols, etc.

On peut avantageusement ajouter ou retirer de l'eau pour ajuster la viscosité de la pâte à extruder. Cette étape peut avantageusement être réalisée à tout stade de l'étape de malaxage.

Pour ajuster la teneur en matière solide de la pâte à extruder afin de la rendre extrudable, on peut également ajouter un composé majoritairement solide et de préférence un oxyde ou un hydrate. On utilise de manière préférée un hydrate et de manière encore plus préférée un hydrate d'aluminium. La perte au feu de cet hydrate est avantageusement supérieure à 15%.

L'extrusion de la pâte issue de l'étape de malaxage peut avantageusement être réalisée par n'importe quel outil conventionnel, disponible commercialement. La pâte issue du malaxage est avantageusement extrudée à travers une filière, par exemple à l'aide d'un piston ou d'une mono-vis ou double vis d'extrusion. L'extrusion peut avantageusement être réalisée par toute méthode connue de l'homme de métier.

Les supports du catalyseur selon l'invention sont en général sous la forme d'extrudés cylindriques ou polylobés tels que bilobés, trilobés, polylobés de forme droite ou torsadée, mais peuvent éventuellement être fabriqués et employés sous la forme de poudres concassées, de tablettes, d'anneaux, de billes et/ou de roues. De préférence, les supports du catalyseur selon l'invention ont la forme de sphères ou d'extrudés. Avantageusement le support se présente sous forme d'extrudés d'un diamètre compris entre 0,5 et 5 mm et plus particulièrement entre 0,7 et 2,5 mm. Les formes peuvent être cylindriques (qui peuvent être creuses ou non) et/ou cylindriques torsadés et/ou multilobées (2, 3, 4 ou 5 lobes par exemple) et/ou anneaux. La forme multilobée est avantageusement utilisée de manière préférée.

Séchage

Le support ainsi obtenu peut ensuite être soumis à une étape de séchage. Ladite étape de séchage est avantageusement effectuée par toute technique connue de l'homme du métier.

De préférence, le séchage est effectué sous flux d'air. Ledit séchage peut également être effectué sous flux de tout gaz oxydant, réducteur ou inerte. De préférence, le séchage est avantageusement effectué à une température comprise entre 50 et 180°C, de manière préférée entre 60 et 150°C et de manière très préférée entre 80 et 130°C.

Calcination

Ledit support, éventuellement séché, subit ensuite de préférence une étape de calcination.

Ladite étape de calcination est avantageusement réalisée en présence d'oxygène moléculaire, par exemple en effectuant un balayage d'air, à une température avantageusement supérieure à 200°C et inférieure ou égale à 1100°C. Ladite étape de calcination peut avantageusement être effectuée en lit traversé, en lit léché ou en atmosphère statique. Par exemple, le four utilisé peut être un four rotatif tournant ou être un four vertical à couches traversées radiales. De préférence, ladite étape de calcination est effectuée entre plus d'une heure à 200°C à moins d'une heure à 1100°C. La calcination peut avantageusement être opérée en présence de vapeur d'eau et/ou en présence d'une vapeur acide ou basique. Par exemple, la calcination peut être réalisée sous pression partielle d'ammoniaque.

Traitements post-calcination

Des traitements post-calcination peuvent éventuellement être effectués, de manière à améliorer les propriétés du support, notamment les propriétés texturales.

Ainsi, le support du catalyseur mis en œuvre dans le procédé selon la présente invention peut être soumis à un traitement hydrothermal en atmosphère confinée. On entend par traitement hydrothermal en atmosphère confinée un traitement par passage à l'autoclave en présence d'eau à une température supérieure à la température ambiante, de préférence supérieure à 25°C, de préférence supérieure à 30°C.

Au cours de ce traitement hydrothermal, on peut avantageusement imprégner le support, préalablement à son passage à l'autoclave (l'autoclavage étant fait soit en phase vapeur, soit en phase liquide, cette phase vapeur ou liquide de l'autoclave pouvant être acide ou non). Cette imprégnation, préalable à l'autoclavage, peut avantageusement être acide ou non. Cette imprégnation, préalable à l'autoclavage peut avantageusement être effectuée à sec ou par immersion du support dans une solution aqueuse acide. Par imprégnation à sec, on entend mise en contact du support avec un volume de solution inférieur ou égal au volume poreux total du support. De préférence, l'imprégnation est réalisée à sec. L'autoclave est de préférence un autoclave à panier rotatif tel que celui défini dans la demande brevet EP 0 387 109 A. La température pendant l'autoclavage peut être comprise entre 100 et 250°C pendant une période de temps comprise entre 30 minutes et 3 heures.

Dépôt de la phase métallique

Pour le dépôt du métal du groupe VIII de la classification périodique des éléments, toutes les techniques de dépôt connues de l'homme du métier et tous les précurseurs de tels métaux peuvent convenir. On peut utiliser des techniques de dépôt par imprégnation à sec ou en excès d'une solution contenant les précurseurs des métaux, en présence de compétiteurs ou non. L'introduction du métal peut s'effectuer à toute étape de la préparation du catalyseur: sur la zéolithe IZM-2 et/ou sur la matrice, notamment avant l'étape de mise en forme, pendant l'étape de mise en forme, ou après l'étape de mise en forme, sur le support du catalyseur. De manière préférée le dépôt du métal s'effectue après l'étape de mise en forme.

Le contrôle de certains paramètres mis en œuvre lors du dépôt, en particulier la nature du précurseur du (des) métal(ux) du groupe VIII utilisé(s), permet d'orienter le dépôt du(es)dit(s) métal(ux) majoritairement sur la matrice ou sur la zéolithe.

Ainsi, pour introduire le(s) métal(ux) du groupe VIII, préférentiellement le platine et/ou le palladium, majoritairement sur la matrice, on peut mettre en œuvre un échange anionique avec de l'acide hexachloroplatinique et/ou de l'acide hexachloropalladique, en présence d'un agent compétiteur, par exemple de l'acide chlorhydrique, le dépôt étant en général suivi d'une calcination, par exemple à une température comprise entre 350 et 550°C et pendant une durée comprise entre 1 et 4 heures. Avec de tels précurseurs, le(s) métal(ux) du groupe VIII est(sont) déposé(s) majoritairement sur la matrice et le(s)dit(s) métal(ux) présente(nt) une bonne dispersion et une bonne répartition macroscopique à travers le grain de catalyseur.

On peut aussi envisager de déposer le(s) métal(ux) du groupe VIII, préférentiellement le platine et/ou le palladium, par échange cationique de manière à ce que le(s)dit(s) métal(ux) soi(en)t déposé(s) majoritairement sur la zéolithe. Ainsi, dans le cas du platine, le précurseur peut être par exemple choisi parmi :

- les composés ammoniaqués tels que les sels de platine (II) tétramines de formule $Pt(NH_3)_4X_2$, les sels de platine (IV) hexamines de formule $Pt(NH_3)_6X_4$ ; les sels de platine (IV) halogénopentamines de formule $(PtX(NH_3)_5)X_3$ ; les sels de platine N-tétrahalogénodiamines de formule $PtX_4(NH_3)_2$ ; et

- les composés halogénés de formule $H(Pt(aca_C)_2X)$;

X étant un halogène choisi dans le groupe formé par le chlore, le fluor, le brome et l'iode, X étant de préférence le chlore, et "acac" représentant le groupe acétylacétonate (de formule brute $C_5H_7O_2$), dérivé de l'acétylacétone. Avec de tels précurseurs, le(s) métal(ux) du groupe VIII est(sont) déposé(s) majoritairement sur la zéolithe et le(s)dit(s) métal(ux) présente(nt) une bonne dispersion et une bonne répartition macroscopique à travers le grain de catalyseur.

Dans le cas où le catalyseur de l'invention contient également au moins un métal choisi parmi les métaux des groupes IIIA, IVA et VIIB, toutes les techniques de dépôt d'un tel métal connues de l'homme du métier et tous les précurseurs de tels métaux peuvent convenir.

On peut ajouter le(s) métal(ux) du groupe VIII et celui(ceux) des groupes IIIA, IVA et VIIB, soit séparément soit simultanément dans au moins une étape unitaire. Lorsqu'au moins un métal des groupes IIIA, IVA et VIIB est ajouté séparément, il est préférable qu'il soit ajouté après le métal du groupe VIII.

Le métal additionnel choisi parmi les métaux des groupes IIIA, IVA et VIIB peut être introduit par l'intermédiaire de composés tels que par exemple les chlorures, les bromures et les nitrates des métaux des groupes IIIA, IVA et VIIB. Par exemple dans le cas de l'indium, on utilise avantageusement le nitrate ou le chlorure et dans le cas du rhénium, on utilise avantageusement l'acide perrhénique. Le métal additionnel choisi parmi les métaux des groupes IIIA, IVA et VIIB peut également être introduit sous la forme d'au moins un composé organique choisi dans le groupe constitué par les complexes dudit métal, en particulier les complexes polycétoniques du métal et les hydrocarbylmétaux tels que les alkyles, les cycloalkyles, les aryles, les alkylaryles et les arylalkyles de métaux. Dans ce dernier cas, l'introduction du métal est avantageusement effectuée à l'aide d'une solution du composé organométallique dudit métal dans un solvant organique. On peut également employer des composés organohalogénés du métal. Comme composés organiques de métaux, on peut citer en particulier le tétrabutylétain, dans le cas de l'étain, et le triphénylindium, dans le cas de l'indium.

Si le métal additionnel choisi parmi les métaux des groupes IIIA, IVA et VIIB est introduit avant le métal du groupe VIII, le composé du métal IIIA, IVA et/ou VIIB utilisé est généralement choisi dans le groupe constitué par l'halogénure, le nitrate, l'acétate, le tartrate, le carbonate et l'oxalate du métal. L'introduction est alors avantageusement effectuée en solution aqueuse. Mais il peut également être introduit à l'aide d'une solution d'un composé organométallique du métal par exemple le tétrabutylétain. Dans ce cas, avant de procéder à l'introduction d'au moins un métal du groupe VIII, on procédera à une calcination sous air.

De plus, des traitements intermédiaires tels que par exemple une calcination et/ou une réduction peuvent être appliqués entre les dépôts successifs des différents métaux.

Avant son utilisation dans le procédé selon l'invention, le catalyseur est de préférence réduit. Cette étape de réduction est avantageusement réalisée par un traitement sous hydrogène à une température comprise entre 150°C et 650°C et une pression totale comprise entre 0,1 et 25 MPa. Par exemple, une réduction consiste en un palier à 150°C de deux heures puis une montée en température jusqu'à 450°C à la vitesse de 1°C/min puis un palier de deux heures à 450°C; durant toute cette étape de réduction, le débit d'hydrogène est de 1000 normaux m$^3$ d'hydrogène par tonne catalyseur et la pression totale maintenue constante à 0,2 MPa. Toute méthode de réduction ex-situ peut avantageusement être envisagée. Une réduction préalable du catalyseur final ex situ, sous courant d'hydrogène, peut être mise en œuvre, par exemple à une température de 450°C à 600°C, pendant une durée de 0,5 à 4 heures.

Ledit catalyseur comprend également avantageusement du soufre. Dans le cas où le catalyseur de l'invention contient du soufre, celui-ci peut être introduit à n'importe quelle étape de la préparation du catalyseur: avant ou après étape de mise en forme, et/ou séchage et/ou calcination, avant et/ou après l'introduction du ou des métaux cités précédemment, ou encore par sulfuration in situ et ou ex situ avant la réaction catalytique. Dans le cas d'une sulfuration in situ, la réduction, si le catalyseur n'a pas été préalablement réduit, intervient avant la sulfuration. Dans le cas d'une sulfuration ex situ, on effectue également la réduction puis la sulfuration. La sulfuration s'effectue de préférence en présence d'hydrogène en utilisant tout agent sulfurant bien connu de l'homme de métier, tel que par exemple le sulfure de diméthyle ou le sulfure d'hydrogène.

Les catalyseurs selon l'invention se présentent sous différentes formes et dimensions. Ils sont utilisés en général sous la forme d'extrudés cylindriques et/ou polylobés tels que bilobés, trilobés, polylobés de forme droite et/ou torsadée, mais peuvent éventuellement être fabriqués et employés sous la forme de poudres concassées, de tablettes, d'anneaux, de billes et/ou de roues. De préférence, les catalyseurs mis en œuvre dans le procédé selon l'invention ont la forme de sphères ou d'extrudés. Avantageusement le catalyseur se présente sous forme d'extrudés d'un diamètre compris entre 0,5 et 5 mm et plus particulièrement entre 0,7 et 2,5 mm. Les formes peuvent être cylindriques (qui peuvent être creuses ou non) et/ou cylindriques torsadés et/ou multilobées (2, 3, 4 ou 5 lobes par exemple) et/ou anneaux. La forme multilobée est avantageusement utilisée de manière préférée. Le dépôt du métal ne change pas la forme du support.

Ledit catalyseur selon l'invention comprend plus particulièrement, et de préférence est constitué de:

- de 1 à 90%, de préférence de 3 à 80% et de manière plus préférée de 4 à 60% et de manière encore plus préférée de 6 à 50% poids de la zéolithe IZM-2 selon l'invention,

- de 0,01 et 5% en poids de manière préférée entre 0,1 et 4% en poids et de manière très préférée entre 0,1 et 2% en poids d'au moins un métal du groupe VIII de la classification périodique des éléments, de préférence le platine,

- éventuellement de 0,01 à 2%, de préférence de 0,05 à 1% poids d'au moins un métal additionnel choisi dans le groupe formé par les métaux des groupes IIIA, IVA et VIIB,

- éventuellement une teneur en soufre, de préférence telle que le rapport du nombre de moles de soufre sur le nombre de moles de(s) métal(ux) du groupe VIII soit compris entre 0,3 et 3,

- au moins une matrice, de préférence l'alumine, assurant le complément à 100% dans le catalyseur.

Les exemples suivants illustrent l'invention sans toutefois en limiter la portée.

**Exemple 1 (non conforme à l'invention)** : préparation du catalyseur A.

Synthèse de la zéolithe IZM-2

781 mg d'une zéolithe de type structural FAU (CBV720, Zeolyst) ont été mélangés avec 6076,6 mg d'une solution aqueuse de dibromure de 1,6-bis (méthylpiperidinium) hexane (20,04 % en poids). 4203 mg d'eau déionisée sont ajoutés au mélange précédent, la préparation obtenue est maintenue sous agitation pendant 10 minutes. 548 mg d'une solution aqueuse à 20% en poids d'hydroxyde de sodium (98% en poids, Aldrich) sont ajoutés. Afin de favoriser la formation du solide cristallisé IZM-2, 103 mg de germes de zéolithe IZM-2 sont ajoutés dans le mélange de synthèse et maintenu sous agitation pendant 15 minutes. Par la suite, 610,3 mg de silice colloïdale (Ludox HS40, 40% en poids, Aldrich) ont été incorporée dans le mélange de synthèse, celui-ci est maintenu sous agitation le temps nécessaire pour évaporer le solvant jusqu'à obtenir la concentration du gel désirée, c'est-à-dire une composition molaire du mélange suivante . 1 $SiO_2$, 0,025 $Al_2O_3$, 0,17 dibromure de 1,6bis(méthylpiperidinium)hexane, 0,0835 $Na_2O$, 33,33 $H_2O$. Le mélange est ensuite transféré, après homogénéisation, dans un autoclave. L'autoclave est fermé puis chauffé pendant 6 jours à 170°C sous agitation. Le produit cristallisé obtenu est filtré, lavé à l'eau déionisée puis séché une nuit à 100°C. Le solide est ensuite introduit dans un four à moufle où est réalisée une étape de calcination : le cycle de calcination comprend une montée en température jusqu'à 200°C, un palier à 200°C maintenu durant 2 heures, une montée en température jusqu'à 550°C suivi d'un palier à 550°C maintenu durant 8 heures puis un retour à la température ambiante. La synthèse est reproduite plusieurs fois afin de disposer d'une quantité suffisante de solide pour les caractérisations et les étapes ultérieures de préparation. Le solide ainsi obtenu est ensuite mis sous reflux durant 2 heures dans une solution aqueuse de nitrate d'ammonium (10 ml de solution par gramme de solide, concentration en nitrate d'ammonium de 3 M) afin d'échanger les cations alcalins sodium par des ions ammonium. Cette étape de mise sous reflux est effectuée quatre fois, puis le solide est filtré, lavé à l'eau déionisée et séché en étuve une nuit à 100°C. Enfin, pour obtenir la zéolithe sous sa forme protonique (acide) on réalise une étape de calcination à 550°C durant dix heures (rampe de montée en température de 5°C/min) en lit traversé sous air sec (2 normaux litres par heure et par gramme de solide). Le solide ainsi obtenu a été analysé par Diffraction des Rayons X et identifié comme étant constitué par de la zéolithe IZM-2. Des caractérisations par RMN de l'$^{27}$Al, fluorescence X et absorption atomique permettent d'accéder aux résultats suivants pour l'IZM-2 :

- pourcentage pondéral d'atomes d'aluminium hexacoordinés Al$^{VI}$ : 8%,

- rapport du nombre de moles de silicium divisé par le nombre de moles d'aluminium de réseau, en mol/mol, Si/Al : 22

- rapport du nombre de moles de sodium divisé par le nombre de moles d'aluminium de réseau, en mol/mol, Na/Al : 0,02.

Préparation du support IZM-2/alumine.

Le support IZM-2/alumine est obtenu par malaxage et extrusion de la zéolithe IZM-2 avec un gel d'alumine de type GA7001 fourni par la société AXENS. La pâte malaxée est extrudée au travers d'une filière trilobes de diamètre 1,8 mm. Après séchage en étuve une nuit à 110°C, les extrudés sont calcinés à 500°C durant deux heures (rampe de montée en température de 5°C/min) en lit traversé sous air sec (2 normaux litres par heure et par gramme de solide). La teneur pondérale de la zéolithe IZM-2 sur le support après calcination est de 25% poids.

Dépôt du platine.

Le platine est déposé par imprégnation en excès du support IZM-2/alumine par une solution aqueuse contenant de l'acide hexachloroplatinique. La concentration en acide hexachloroplatinique dans la solution est de 2,55 $10^{-3}$ mol/l. On utilise 20 grammes de support dont on remplit le volume poreux par de l'eau distillée et on laisse le solide à maturer durant une heure à température ambiante. Le solide est ensuite immergé dans 80 ml d'une solution d'acide chlorhydrique HCl de concentration 3,52 $10^{.1}$ mol/l dans un erlenmeyer puis l'ensemble est mis sur agitation sur une table d'agitation (100 tours/min) à température ambiante durant une heure. La solution d'acide chlorhydrique est ensuite soutirée puis le solide est immergé dans 80 ml de la solution d'acide hexachloroplatinique précédemment décrite puis l'ensemble est mis sur agitation sur une table d'agitation (100 tours/min) à température ambiante durant 24 heures. La solution d'imprégnation est ensuite soutirée et le solide est rincé avec 160 ml d'eau distillée. Le solide est ensuite mis à sécher en étuve ventilée durant la nuit à 110°C et on effectue finalement une étape de calcination sous débit d'air sec (2 normaux litres par heure et par gramme de solide) dans un four tubulaire dans les conditions suivantes :

- montée de la température à l'ambiante à 500°C à 5°C/min ;

- palier de deux heures à 500°C ;

- descente à l'ambiante.

La teneur en Pt mesurée par FX sur le catalyseur calciné est de 0,19% en poids, sa dispersion mesurée par titrage $H_2/O_2$ est de 85%, son coefficient de répartition mesuré par microsonde de Castaing de 0,94.

**Exemple 2 (conforme à l'invention)** : préparation du catalyseur B.

Synthèse de la zéolithe IZM-2

Cette zéolithe IZM-2 a été synthétisée conformément à l'enseignement de la demande de brevet FR 2 918 050. Une suspension colloïdale de silice connue sous le terme commercial Ludox HS-40 commercialisée par Aldrich, est incorporée dans une solution composée de soude (Prolabo), de structurant dibromure de 1,6bis(méthylpiperidinium)hexane, d'hydroxyde d'aluminium (Aldrich) et d'eau déionisée. La composition molaire du mélange est la suivante : 1 $SiO_2$, 0,0166 $Al_2O_3$, 0,1666 $Na_2O$, 0,1666 1,6bis(méthylpiperidinium)hexane, 33,3333 $H_2O$. Le mélange est agité vigoureusement pendant un demi-heure. Le mélange est ensuite transféré, après homogénéisation, dans un autoclave de type PARR. L'autoclave est chauffé pendant 5 jours à 170°C sous agitation en tourne broche (30 tours/min). Le produit obtenu est filtré, lavé à l'eau déionisée pour atteindre un pH neutre puis séché une nuit à 100°C en étuve. Le solide est ensuite introduit dans un four à moufle pour y être calciné afin d'éliminer le structurant. Le cycle de calcination comprend une montée en température jusqu'à 200°C, un palier à cette température de deux heures, une montée en température jusqu'à 550°C suivi d'un palier de huit heures à cette température et enfin un retour à température ambiante. Les montées en température sont effectuées avec une rampe de 2°C/min. Le solide ainsi obtenu est ensuite mis sous reflux durant 2 heures dans une solution aqueuse de nitrate d'ammonium (10 ml de solution par gramme de solide, concentration en nitrate d'ammonium de 3 M) afin d'échanger les cations alcalins sodium par des ions ammonium. Cette étape de mise sous reflux est effectuée quatre fois, puis le solide est filtré, lavé à l'eau déionisée et séché en étuve une nuit à 100°C. Enfin, pour obtenir la zéolithe sous sa forme protonique (acide) on réalise une étape de calcination à 550°C durant dix heures (rampe de montée en température de 5°C/min) en lit traversé sous air sec (2 normaux litres par heure et par gramme de solide). Le solide ainsi obtenu a été analysé par Diffraction des Rayons X et identifié comme étant constitué par de la zéolithe IZM-2. Des caractérisations par RMN de l'$^{27}$Al, fluorescence X et absorption atomique permettent d'accéder aux résultats suivants pour l'IZM-2 :

- pourcentage pondéral d'atomes d'aluminium hexacoordinés $Al^{VI}$ : 7%,

- rapport du nombre de moles de silicium divisé par le nombre de moles d'aluminium de réseau, en mol/mol, Si/Al : 32,

- rapport du nombre de moles de sodium divisé par le nombre de moles d'aluminium de réseau, en mol/mol, Na/Al : 0,07.

Préparation du support IZM-2/alumine.

Le support IZM-2/alumine est obtenu selon le même procédé que celui décrit dans l'exemple 1.

Dépôt du platine.

Le platine est déposé par imprégnation en excès du support IZM-2/alumine selon le même procédé que celui décrit dans l'exemple 1.

La teneur en Pt mesurée par FX sur le catalyseur calciné est de 0,20% en poids, sa dispersion mesurée par titrage $H_2/O_2$ est de 83%, son coefficient de répartition mesuré par microsonde de Castaing de 0,98.

**Exemple 3 (conforme à l'invention)** : préparation du catalyseur C.

Synthèse de la zéolithe IZM-2

Cette zéolithe IZM-2 a été synthétisée conformément à l'enseignement de la demande de brevet FR 2 918 050. Une suspension colloïdale de silice connue sous le terme commercial Ludox HS-40 commercialisée par Aldrich, est incorporée dans une solution composée de soude (Prolabo), de structurant dibromure de 1,6bis(méthylpiperidinium)hexane, d'hydroxyde d'aluminium (Aldrich) et d'eau déionisée. La composition molaire du mélange est la suivante : 1 $SiO_2$, 0,0100 $Al_2O_3$, 0,1666 $Na_2O$, 0,1666 1,6bis(méthylpiperidinium)hexane, 33,3333 $H_2O$. Le mélange est agité vigoureusement pendant un demi-heure. Le mélange est ensuite transféré, après homogénéisation, dans un autoclave de type PARR. L'autoclave est chauffé pendant 5 jours à 170°C sous agitation en tourne broche (30 tours/min). Le produit obtenu est filtré, lavé à l'eau déionisée pour atteindre un pH neutre puis séché une nuit à 100°C en étuve. Le solide est ensuite introduit dans un four à moufle pour y être calciné afin d'éliminer le structurant. Le cycle de calcination comprend une montée en température jusqu'à 200°C, un palier à cette température de deux heures, une montée en température jusqu'à 550°C suivi d'un palier de huit heures à cette température et enfin un retour à température ambiante. Les montées en température sont effectuées avec une rampe de 2°C/min. Le solide ainsi obtenu est ensuite mis sous reflux durant 2 heures dans une solution aqueuse de nitrate d'ammonium (10 ml de solution par gramme de solide, concentration en nitrate d'ammonium de 3 M) afin d'échanger les cations alcalins sodium par des ions ammonium. Cette étape de mise sous reflux est effectuée quatre fois, puis le solide est filtré, lavé à l'eau déionisée et séché en étuve une nuit à 100°C. Enfin, pour obtenir la zéolithe sous sa forme protonique (acide) on réalise une étape de calcination à 550°C durant dix heures (rampe de montée en température de 5°C/min) en lit traversé sous air sec (2 normaux litres par heure et par gramme de solide). Le solide ainsi obtenu a été analysé par Diffraction des Rayons X et identifié comme étant constitué par de la zéolithe IZM-2. Des caractérisations par RMN de l'$^{27}$Al, fluorescence X et absorption atomique permettent d'accéder aux résultats suivants pour l'IZM-2 :

- pourcentage pondéral d'atomes d'aluminium hexacoordinés $Al^{VI}$ : 6%,

- rapport du nombre de moles de silicium divisé par le nombre de moles d'aluminium de réseau, en mol/mol, Si/Al : 46,

- rapport du nombre de moles de sodium divisé par le nombre de moles d'aluminium de réseau, en mol/mol, Na/Al : 0,08.

Préparation du support IZM-2/alumine.

Le support IZM-2/alumine est obtenu selon le même procédé que celui décrit dans l'exemple 1.

Dépôt du platine.

Le platine est déposé par imprégnation en excès du support IZM-2/alumine selon le même procédé que celui décrit dans l'exemple 1.
La teneur en Pt mesurée par FX sur le catalyseur calciné est de 0,20% en poids, sa dispersion mesurée par titrage $H_2/O_2$ est de 83%, son coefficient de répartition mesuré par microsonde de Castaing de 0,98.

**Exemple 4 (non conforme à l'invention)** : préparation du catalyseur D.

Synthèse de la zéolithe IZM-2

La zéolithe IZM-2 a été synthétisée conformément à l'enseignement du brevet FR 2 918 050 B. Une suspension colloïdale de silice connue sous le terme commercial Ludox HS-40 commercialisée par Aldrich, est incorporée dans une solution composée de soude (Prolabo), de structurant dibromure de 1,6bis(méthylpiperidinium)hexane, d'hydroxyde d'aluminium (Aldrich) et d'eau déionisée. La composition molaire du mélange est la suivante : 1 $SiO_2$, 0,0076 $Al_2O_3$, 0,1666 $Na_2O$, 0,1666 1,6bis(méthylpiperidinium)hexane, 33,3333 $H_2O$. Le mélange est agité vigoureusement pendant une demi-heure. Le mélange est ensuite transféré, après homogénéisation, dans un autoclave de type PARR. L'autoclave est chauffé pendant 5 jours à 170°C sous agitation en tourne broche (30 tours/min). Le produit obtenu est filtré, lavé à l'eau déionisée pour atteindre un pH neutre puis séché une nuit à 100°C en étuve. La zéolithe IZM-2 brute de synthèse subit alors une calcination à 550°C durant dix heures (rampe de montée en température de 5°C/min) en lit traversé sous air sec (2 normaux litres par heure et par gramme de solide). Le solide obtenu est mis sous reflux durant 4 heures dans une solution de nitrate d'ammonium (10 ml de solution par gramme de solide, concentration en chlorure d'ammonium de 10 M) afin d'échanger les cations alcalins par des ions ammonium. Cette étape de mise sous reflux est effectuée quatre fois. Le solide est ensuite filtré, lavé à l'eau déionisée et séché en étuve une nuit à 100°C. Enfin, pour obtenir la zéolithe sous sa forme protonique (acide) on réalise une étape de calcination à 550°C durant dix heures (rampe de montée en température de 5°C/min) en lit traversé sous air sec (2 normaux litres par heure et par gramme de solide). Le solide ainsi obtenu a été analysé par Diffraction des Rayons X et identifié comme étant constitué par de la zéolithe IZM-2. Des caractérisations par RMN de l'$^{27}$Al, fluorescence X et absorption atomique permettent d'accéder aux résultats suivants pour l'IZM-2 :

- pourcentage pondéral d'atomes d'aluminium hexacoordinés Al$^{VI}$ : 8%,

- rapport du nombre de moles de silicium divisé par le nombre de moles d'aluminium de réseau, en mol/mol, Si/Al : 58,

- rapport nombre de moles de silicium divisé par le nombre de moles d'aluminium global en mol/mol mesuré par Fluorescence X : 53

- rapport du nombre de moles de sodium divisé par le nombre de moles d'aluminium de réseau, en mol/mol, Na/Al : 0,05.

Préparation du support IZM-2/alumine.

Le support IZM-2/alumine est obtenu selon le même procédé que celui décrit dans l'exemple 1.

Dépôt du platine.

Le platine est déposé par imprégnation en excès du support IZM-2/alumine selon le même procédé que celui décrit dans l'exemple 1.
La teneur en Pt mesurée par FX sur le catalyseur calciné est de 0,21% en poids, sa dispersion mesurée par titrage H$_2$/O$_2$ est de 80%, son coefficient de répartition mesuré par microsonde de Castaing de 0,97.

Exemple 5 (non conforme à l'invention) : préparation du catalyseur E.

Synthèse de la zéolithe IZM-2

La zéolithe IZM-2 a été synthétisée conformément à l'enseignement de la demande de brevet FR 2 918 050 A. Une suspension colloïdale de silice connue sous le terme commercial Ludox HS-40 commercialisée par Aldrich, est incorporée dans une solution composée de soude (Prolabo), de structurant dibromure de 1,6bis(méthylpiperidinium)hexane, d'hydroxyde d'aluminium (Aldrich) et d'eau déionisée. La composition molaire du mélange est la suivante : 1 SiO$_2$, 0,0060 Al$_2$O$_3$, 0,1666 Na$_2$O, 0,1666 dibromure de 1,6bis(méthylpiperidinium)hexane, 33,3333 H$_2$O. Le mélange est agité vigoureusement pendant une demi-heure. Le mélange est ensuite transféré, après homogénéisation, dans un autoclave de type PARR. L'autoclave est chauffé pendant 5 jours à 170°C sous agitation en tourne broche (30 tours/min). Le produit obtenu est filtré, lavé à l'eau déionisée pour atteindre un pH neutre puis séché une nuit à 100°C en étuve. Le solide est ensuite introduit dans un four à moufle pour y être calciné afin d'éliminer le structurant. Le cycle de calcination comprend une montée en température jusqu'à 200°C, un palier à cette température de deux heures, une montée en température jusqu'à 550°C suivi d'un palier de huit heures à cette température et enfin un retour à température ambiante. Les montées en température sont effectuées avec une rampe de 2°C/min. Le solide ainsi obtenu est ensuite mis sous reflux durant 2 heures dans une solution aqueuse de nitrate d'ammonium (10 ml de solution par gramme de solide, concentration en nitrate d'ammonium de 3 M) afin d'échanger les cations alcalins sodium par des ions ammonium. Cette étape de mise sous reflux est effectuée quatre fois avec une solution fraiche de nitrate d'ammonium, puis le solide est filtré, lavé à l'eau déionisée et séché en étuve une nuit à 100°C. Enfin, pour obtenir la zéolithe sous sa forme acide (protonée H+) on réalise une étape de calcination à 550°C durant dix heures (rampe de montée en température de 2°C/min) en lit traversé sous air sec (2 normaux litres par heure et par gramme de solide). Le solide ainsi obtenu a été analysé par Diffraction des Rayons X et identifié comme étant constitué par de la zéolithe IZM-2. Des caractérisations au moyen des méthodes RMN de l'$^{27}$Al, fluorescence X et ICP permettent d'accéder aux résultats suivants pour l'IZM-2 :

- pourcentage pondéral d'atomes d'aluminium hexacoordinés Al$^{VI}$ : 5%,

- rapport du nombre de moles de silicium divisé par le nombre de moles d'aluminium de réseau, en mole/mole, Si/Al : 72,

- rapport du nombre de moles de sodium divisé par le nombre de moles d'aluminium de réseau, en mole/mole, Na/Al : 0,03.

Préparation du support IZM-2/alumine.

Le support IZM-2/alumine est obtenu selon le même procédé que celui décrit dans l'exemple 1.

Dépôt du platine.

Le platine est déposé par imprégnation en excès du support IZM-2/alumine selon le même procédé que celui décrit dans l'exemple 1.

La teneur en Pt mesurée par FX sur le catalyseur calciné est de 0,20% en poids, sa dispersion mesurée par titrage $H_2/O_2$ est de 83%, son coefficient de répartition mesuré par microsonde de Castaing de 0,95.

Exemple 6 : évaluation des propriétés catalytiques des catalyseurs B et C conformes à l'invention et A. D et E non conformes à l'invention, en isomérisation d'une charge paraffinique.

Les catalyseurs ont été testés en isomérisation d'une charge paraffinique composée par du n-hexadécane. Les tests ont été effectués dans une micro-unité mettant en œuvre un réacteur lit fixe et travaillant en courant descendant sans recyclage. L'analyse des effluents hydrocarbonés est effectuée en ligne par chromatographie en phase gazeuse. Une fois chargé dans l'unité, le catalyseur subit une première étape de séchage sous azote dans les conditions suivantes :

- débit d'azote: 2 normaux litres par heure et par gramme de catalyseur,

- pression totale: 0,1 MPa,

- rampe de montée en température de l'ambiante à 150°C: 5°C/min,

- palier à 150°C de 30 minutes.

Après séchage l'azote est remplacé par l'hydrogène et une étape de réduction sous débit d'hydrogène pur est effectuée ensuite dans les conditions suivantes:

- débit d'hydrogène: 5 normaux litres par heure et par gramme de catalyseur,

- pression totale: 1,1 MPa,

- rampe de montée en température de 150 à 450°C: 5°C/min,

- palier à 450°C de 1 heure.

Après étape de réduction, la température est descendue à 230°C, et le catalyseur est mis en contact du n-hexadécane dans les conditions suivantes :

- vitesse spatiale horaire de 2 grammes de n-hexadécane par heure et par gramme de catalyseur,

- pression partielle en hydrogène de 1,0 MPa

- pression totale de 1,1 MPa.

La conversion est modifiée en faisant varier la température; et à chaque palier de température deux analyses de l'effluent sont effectuées, ce qui permet de calculer les performances catalytiques et de vérifier la stabilité des performances catalytiques pour ledit palier de température. Typiquement on fait varier la température entre 230 et 350°C par palier de température de 5°C. L'analyse des effluents s'effectue intégralement par le biais d'un système GC en ligne. La température nécessaire pour atteindre 50% de conversion fait office de descripteur de l'activité du catalyseur alors que le rendement maximal obtenu en isomères de l'hexadécane fait office de descripteur des propriétés isomérisantes du catalyseur.

**Tableau 1** : performances catalytiques des catalyseurs A, B, C, D et E en hydroconversion du n-hexadécane.

| Catalyseur | A (non conforme) | B (conforme) | C (conforme) | D (non conforme) | E (non conforme) |
|---|---|---|---|---|---|
| Température à 50% conversion (°C) | 233 | 253 | 258 | 262 | 269 |
| Rendement max en isomères (%poids) | 57 | 84 | 85 | 85 | 84 |

On constate que les catalyseurs B et C conformes présentent des propriétés isomérisantes comparables et mais des activités plus élevées que celles des catalyseurs D et E non conformes. Les catalyseurs B et C conformes présentent des activités moins importantes mais des propriétés isomérisantes plus importantes que celles du catalyseur A non conforme.

**Revendications**

1.  Procédé d'isomérisation de charges paraffiniques comprenant une teneur en n-paraffines supérieure à 70% poids par rapport à la masse totale de ladite charge, les paraffines de ladite charge paraffinique présentant un nombre d'atomes de carbone compris entre 9 et 25, ladite charge paraffinique étant produite à partir de ressources renouvelables choisies parmi les huiles végétales, les huiles d'algues ou algales, les huiles de poissons et les graisses d'origine végétale ou animale, ou des mélanges de telles charges, ou par un procédé mettant en jeu une étape de valorisation par la voie Fischer-Tropsch, opérant à une température comprise entre 200°C et 500°C, à une pression totale comprise entre 0,45 MPa et 7 MPa, à une pression partielle d'hydrogène comprise entre 0,3 et 5,5 MPa, à une vitesse spatiale horaire comprise entre 0,1 et 10 kilogramme de charge introduite par kilogramme de catalyseur et par heure et mettant en œuvre un catalyseur comprenant au moins un métal du groupe VIII de la classification périodique des éléments, au moins une matrice et au moins une zéolithe IZM-2, dans lequel le rapport entre le nombre de moles de silicium et le nombre de moles d'aluminium du réseau de la zéolithe IZM-2 est compris entre 25 et 55.

2.  Procédé selon la revendication 1 dans lequel le rapport entre le nombre de moles de silicium et le nombre de moles d'aluminium du réseau de la zéolithe IZM-2 est compris entre 25 et 50.

3.  Procédé selon la revendication 2 dans lequel le rapport entre le nombre de moles de silicium et le nombre de moles d'aluminium du réseau de la zéolithe IZM-2 est compris entre 30 et 50.

4.  Procédé selon l'une des revendications 1 à 3 dans lequel ladite matrice est choisie dans le groupe formé par l'alumine, la silice, la silice-alumine, les argiles, l'oxyde de titane, l'oxyde de bore et la zircone, pris seuls ou en mélange ou bien des aluminates.

5.  Procédé selon l'une des revendications précédentes dans lequel le métal du groupe VIII est choisi parmi le fer, le cobalt, le nickel, le ruthénium, le rhodium, le palladium, l'osmium, l'iridium et le platine.

6.  Procédé selon la revendication 5 dans lequel le métal du groupe VIII est choisi parmi les métaux nobles du groupe VIII choisis parmi le palladium et le platine.

7.  Procédé selon la revendication 6 dans lequel la teneur en métal noble dudit catalyseur est comprise entre 0,01 et 5% poids par rapport à la masse totale dudit catalyseur.

8.  Procédé selon l'une des revendications précédentes comprenant au moins un métal additionnel choisi dans le groupe formé par les métaux des groupes IIIA, IVA et VIIB de la classification périodique des éléments et de préférence parmi le gallium, l'indium, l'étain et le rhénium.

9.  Procédé selon l'une des revendications précédentes dans lequel ledit procédé opère à une température comprise entre 200°C et 450°C, à une pression totale comprise entre 0,6 MPa et 6 MPa, à une pression partielle d'hydrogène comprise entre 0,4 et 4,8 MPa, à une vitesse spatiale horaire comprise entre 0,2 et 7 kilogramme de charge introduite par kilogramme de catalyseur et par heure.

## Patentansprüche

1. Verfahren zur Isomerisierung von paraffinischen Einsatzstoffen mit einem Gehalt an n-Paraffinen von mehr als 70 Gew.-%, bezogen auf die Gesamtmasse des Einsatzstoffs, wobei die Paraffine des paraffinischen Einsatzstoffs eine Zahl von Kohlenstoffatomen zwischen 9 und 25 aufweisen, wobei der paraffinische Einsatzstoff aus nachwachsenden Rohstoffen, die aus Pflanzenölen, Ölen aus Algen oder Algenölen, Fischölen und Fetten pflanzlicher oder tierischer Herkunft oder Mischungen derartiger Einsatzstoffe ausgewählt sind, oder durch ein Verfahren mit einem Schritt der Veredelung über die Fischer-Tropsch-Route bei einer Temperatur zwischen 200 °C und 500 °C, einem Gesamtdruck zwischen 0,45 MPa und 7 MPa, einem Wasserstoff-Partialdruck zwischen 0,3 und 5,5 MPa, einer Katalysatorbelastung zwischen 0,1 und 10 Kilogramm eingetragenem Einsatzstoff pro Kilogramm Katalysator und pro Stunde und unter Verwendung eines Katalysators, der mindestens ein Metall der Gruppe VIII des Periodensystems der Elemente, mindestens eine Matrix und mindestens einen Zeolith IZM-2 umfasst, wobei das Verhältnis zwischen der Zahl der Mole Silicium und der Zahl der Mole Aluminium des Netzwerks des Zeoliths IZM-2 zwischen 25 und 55 liegt, hergestellt wird.

2. Verfahren nach Anspruch 1, wobei das Verhältnis zwischen der Zahl der Mole Silicium und der Zahl der Mole Aluminium des Netzwerks des Zeoliths IZM-2 zwischen 25 und 50 liegt.

3. Verfahren nach Anspruch 2, wobei das Verhältnis zwischen der Zahl der Mole Silicium und der Zahl der Mole Aluminium des Netzwerks des Zeoliths IZM-2 zwischen 30 und 50 liegt.

4. Verfahren nach einem der Ansprüche 1 bis 3, wobei die Matrix aus der Gruppe ausgewählt ist, die von Aluminiumoxid, Siliciumdioxid, Siliciumdioxid-Aluminiumoxid, Tonen, Titanoxid, Boroxid und Zirconiumdioxid für sich alleine oder als Mischung oder auch von Aluminaten gebildet wird.

5. Verfahren nach einem der vorhergehenden Ansprüche, wobei das Metall der Gruppe VIII aus Eisen, Cobalt, Nickel, Ruthenium, Rhodium, Palladium, Osmium, Iridium und Platin ausgewählt ist.

6. Verfahren nach Anspruch 5, wobei das Metall der Gruppe VIII aus Edelmetallen der Gruppe VIII, die aus Palladium und Platin ausgewählt sind, ausgewählt ist.

7. Verfahren nach Anspruch 6, wobei der Edelmetallgehalt des Katalysators zwischen 0,01 und 5 Gew.-%, bezogen auf die gesamte Masse des Katalysators, liegt.

8. Verfahren nach einem der vorhergehenden Ansprüche, umfassend mindestens ein zusätzliches Metall, das aus der Gruppe, die von den Metallen der Gruppen IIIA, IVA und VIIB des Periodensystems der Elemente gebildet wird, und vorzugsweise aus Gallium, Indium, Zinn und Rhenium ausgewählt ist.

9. Verfahren nach einem der vorhergehenden Ansprüche, wobei das Verfahren bei einer Temperatur zwischen 200 °C und 450 °C, einem Gesamtdruck zwischen 0, 6 MPa und 6 MPa, einem Wasserstoff-Partialdruck zwischen 0,4 und 4,8 MPa und einer Katalysatorbelastung zwischen 0,2 und 7 Kilogramm eingetragenem Einsatzstoff pro Kilogramm Katalysator und pro Stunde durchgeführt wird.

## Claims

1. Process for the isomerization of paraffinic feedstocks comprising a content of n-paraffins of greater than 70% by weight relative to the total mass of said feedstock, the paraffins of said paraffinic feedstock having a number of carbon atoms of between 9 and 25, said paraffinic feedstock being produced from renewable resources chosen from plant oils, oils of algae or algal oils, fish oils and fats of plant or animal origin, or mixtures of such feedstocks, or by means of a process involving a step of upgrading via the Fischer-Tropsch route, operating at a temperature of between 200°C and 500°C, at a total pressure of between 0.45 MPa and 7 MPa, at a hydrogen partial pressure of between 0.3 and 5.5 MPa, at an hourly space velocity of between 0.1 and 10 kilograms of feedstock introduced per kilogram of catalyst and per hour and using a catalyst comprising at least one metal of group VIII of the periodic table of elements, at least one matrix and at least one zeolite IZM-2, in which the ratio between the number of moles of silicon and the number of moles of aluminium of the zeolite IZM-2 network is between 25 and 55.

2. Process according to Claim 1, in which the ratio between the number of moles of silicon and the number of moles of aluminium of the zeolite IZM-2 network is between 25 and 50.

3. Process according to Claim 2, in which the ratio between the number of moles of silicon and the number of moles of aluminium of the zeolite IZM-2 network is between 30 and 50.

4. Process according to one of Claims 1 to 3, in which said matrix is chosen from the group formed by alumina, silica, silica-alumina, clays, titanium oxide, boron oxide and zirconia, taken alone or as a mixture, or else aluminates.

5. Process according to one of the preceding claims, in which the metal of group VIII is chosen from iron, cobalt, nickel, ruthenium, rhodium, palladium, osmium, iridium and platinum.

6. Process according to Claim 5, in which the metal of group VIII is chosen from the noble metals of group VIII, chosen from palladium and platinum.

7. Process according to Claim 6, in which the noble metal content of said catalyst is between 0.01% and 5% by weight relative to the total weight of said catalyst.

8. Process according to one of the preceding claims, comprising at least one additional metal chosen from the group formed by metals of groups IIIA, IVA and VIIB of the periodic table of elements, preferably chosen from gallium, indium, tin and rhenium.

9. Process according to one of the preceding claims, in which said process is carried out at a temperature of between 200°C and 450°C, at a total pressure of between 0.6 MPa and 6 MPa, at a hydrogen partial pressure of between 0.4 and 4.8 MPa, at an hourly space velocity of between 0.2 and 7 kilograms of feedstock introduced per kilogram of catalyst and per hour.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2918050 A
- FR 2984911 A
- FR 2918050
- FR 2934794
- FR 2910483
- FR 2950895
- EP 0387109 A
- FR 2918050 B

**Littérature non-brevet citée dans la description**

- **C. MARCILLY.** *catalyse acido-basique,* 2003, vol. 1
- **F. ALVAREZ.** *Journal of Catalysis,* 1996, vol. 162, 179
- **P. MENDES.** *AlChE Journal,* 2017, vol. 63 (7), 2864
- Analyse physico-chimiques des catalyseurs industriels. J. Lynch. 2001, 290-291